(19) Europäisches Patentamt
European Patent Office
Office européen des brevets

(11) **EP 3 901 965 A1**

(12) **EUROPEAN PATENT APPLICATION**

(43) Date of publication:
**27.10.2021 Bulletin 2021/43**

(51) Int Cl.:
***G16H 50/30*** *(2018.01)*   ***G16H 50/50*** *(2018.01)*

(21) Application number: **20170425.1**

(22) Date of filing: **20.04.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Masaryk University**
**601 77 Brno (CZ)**

(72) Inventors:
- **DOBROVOLNÁ, Julie**
  **602 00 Brno (CZ)**
- **LENÁRT, Peter**
  **664 84 Zastávka (CZ)**
- **ZLÁMAL, Filip**
  **613 00 Brno (CZ)**

(74) Representative: **Grünecker Patent- und Rechtsanwälte**
**PartG mbB**
**Leopoldstraße 4**
**80802 München (DE)**

(54) **STRESS TWIN FOR INDIVIDUALS**

(57)    The present invention relates to the concept of a stress digital twin. The stress digital twin of an individual is a digital, i.e. computer-implemented system for monitoring a stress level of a living individual on the basis of at least data directly measured on the body of the living individual and by performing a thermodynamic evaluation of the stress entropic load. The stress entropic load is a thermodynamic parameter representing an amount of entropy generated within the living individual due to adaptation to a whole set of environmental influences in a given scenario at a given point of time. Based on a comparison with pre-collected reference data (library data), the invention triggers actions such as withdrawal or replacement of individuals acting in a given scenario or the issuing of alerts.

Fig. 1

EP 3 901 965 A1

**EP 3 901 965 A1**

**Description**

**FIELD OF THE INVENTION**

The present invention relates to a stress digital twin for an individual or a group of individuals. More specifically, the present invention relates to a system and a method for monitoring a stress level of a living individual.

**BACKGROUND**

It is often desirable to assess whether a person is stressed and/or quantify their stress levels. For example, in demanding high-stress jobs, for example, surgeons, flight dispatchers or military positions, etc. it would be highly useful to know that a given individual has stress levels highly exceeding their normal levels which increase the likelihood of fatal or costly mistakes. Such knowledge would be invaluable for preventing errors and dangerous situations in many fields by signaling that a given person needs to rest or adjust their current activity as soon as possible, or need to be replaced. However, current technologies are unable to monitor and evaluate individualized stress levels in real time. For example, if two individuals have the same levels of cortisol in their saliva, it may well be the case that one experiences acute stress and the other does not.

In the present disclosure, stress will be understood in a broad sense as being any reaction of a living individual to a wide range of environmental factors, including physical, chemical and biological ones, that induces an adaptation by the individual. Under adaptation, in the present disclosure any type of reaction to external or internal stimuli that requires energy is to be understood.

Therefore, it would be desirable to provide systems and methods, facilitating a real-time assessment and quantification of individualized stress levels in living systems, for example, in humans.

**SUMMARY**

This is achieved by the features of the independent claims.

According to a first aspect of the present invention, a system for monitoring a stress level of a living individual is provided. The system comprises receiving means for receiving data directly measured by a plurality of sensors on the body of the living individual and processing means for evaluating a stress level of the living individual based on the received data. The evaluating of the stress level includes calculating a stress entropic load. The stress entropic load is a thermodynamic parameter representing an amount of entropy generated within the living individual due to adaptation to a whole set of environmental influences in a given scenario at a given point of time. The system further comprises an output interface for generating an output indicating the stress level of the living individual based on the result of the evaluation by said processing means.

According to a second aspect of the present invention, a computer-implemented method of monitoring a stress level of a living individual is provided. The method comprises the steps of receiving data directly measured by a plurality of sensors on the body of the living individual and evaluating a stress level of the living individual based on the received data. The evaluating of the stress level includes calculating a stress entropic load. The stress entropic load is a thermodynamic parameter representing an amount of entropy generated within the living individual due to adaptation to a whole set of environmental influences in a given scenario at a given point of time. The method further comprises the step of outputting an output indicating the stress level of the living individual based on the result of the evaluation.

It is the particular approach of the present invention to evaluate a stress level by entropy based on thermodynamic calculations on the basis of at least data directly measured on the body of the individual by sensors. A stress level is obtained by calculating an amount of entropy generated within an individual (in particular: a human) due to adaptation. A thermodynamic parameter representing said amount of entropy is called "stress entropic load" (SEL). The stress entropic load plays a central role in the concept of the present invention. The stress level evaluation according to the particular approach of the present invention makes possible a prediction of stress level development of the individual in the future. If necessary, an alert can be issued that the stress level of a monitored individual is about to exceed a predefined threshold, or a time until adaptation failure of the individual may be predicted. In particular, the alert may include an indication to replace the individual in his or her current position.

The output interface may include, in particular, a display device or an acoustic signaling device, without being limited to these. For instance, the output interface may include any interface for forwarding an evaluation result to another, external device.

Preferably, the adaptation of the living individual is predicted for future points of time based on the evaluation result. This may include a prediction of future stress levels. The prediction may be based on extrapolation and/or simulation. In the context of the present disclosure, extrapolation refers basically to the use of individual data for prediction of the behavior of the variable later in time. It does not take into account any amendments to initial conditions, just the starting values in the individual. Simulation, on the other hand, refers to more complex process using amendments of the initial as well as later conditions to alter the outcome.

More preferably, an estimated time to adaptation failure of the living individual is predicted based on the extrapolation and/or simulation. The time to adaptation failure does, in particular, define a time limit for replacement or withdrawal of the living individual operating in the given scenario. Thereby failure of the real living individual ("physical twin") can be prevented.

Still more preferably, the estimated time to adaptation failure is based on a comparison of a unique function of a stress entropic load predicted for future points in time with a given threshold. Such a threshold comparison is also possible for a stress level evaluated for a current point of time, in order to issue an immediate alert, in particular, to immediately replace a person, if necessary.

Still more preferably, the unique function of stress entropic load is based on a predetermined library of reference data. The reference data comprise a collection of data reflecting a failure rate of a population under given stress conditions. Alternatively, a direct threshold comparison of the stress entropic load is possible in the framework of the present invention.

Also preferably, the processing means is adapted to determine that the time limit for replacement or withdrawal has been reached. Further preferably, the output interface is adapted to output an indication (alert) to replace or withdraw the living individual, when the time limit for replacement or withdrawal (adaptation failure time) has been reached. Any indication may be output by a plurality of different technical means including but not limited to acoustic signals, optical indications such as by alarm lights of different color and/or different flashing frequency, or more particular information being displayed on a display device. Any combination of the former or other means of indication is possible within the framework of the present invention as well. Further, in particular situations, when a person to be withdrawn or replaced is performing work by means of technical equipment, the system for monitoring a stress level according to the present disclosure, i.e. the "digital twin", may be communicatively connected to the technical equipment so as to prevent the technical equipment from operating further (for example switching the equipment or predetermined functions thereof off), thereby enforcing the withdrawal or replacement. Such a technical support for the replacement may be combined with an alert or may be triggered only at a later stage, when an initial alert was not followed by withdrawing or replacing the person, or after a second, higher stress threshold was exceeded. Such a kind of technically enforced withdrawal may be of particular interest, for instance, in the context of sports, either recreational or at a competitive level.

Also, in embodiments, it is possible to communicatively connect the system for monitoring the stress level of a living individual according to the present invention with technical equipment related to or connected to said individual, for changing settings of said technical equipment based on a stress level indication output by the system. For instance, in a patient undergoing mechanical ventilation, certain patterns of SEL, i.e. stress level, may indicate a bad prognosis and this prognosis could be improved by changing settings of the mechanical ventilation equipment. These changes could be done automatically and in real time, triggered by the system according to the present invention communicatively coupled to the mechanical ventilation equipment.

In accordance with preferred embodiments of the present invention, the receiving means further receives data directly measured by at least one environment sensor. In the embodiments, environmental data measured by environment sensors are further used in the evaluation of the stress level.

Preferably, the calculation of the stress entropic load comprises the calculation of a stress entropic load change of the living individual during a predetermined time interval, i.e. a time interval of a predetermined length. The stress entropic load change is calculated as the difference between the overall entropy production of the living individual during a time interval of a predetermined length and a baseline entropy production of the living individual over the same time interval. The baseline entropy production indicates the entropy production of the living individual during a time interval of the predetermined length. A processing like this ensures that the result reflects the entropy production due to stress only, whereas the measured (internal and possibly also external) thermodynamic parameters of the body do not separately reflect changes due to stress.

More preferably, the baseline entropy production is calculated in advance, by calculating the overall entropy production of the living individual in the time interval of the predetermined length in a stress free scenario, based on respective data measured in the stress free scenario. Still more preferably, data calculated in advance and representing the baseline entropy production for a given living individual are pre-stored in a database. This collection of data is also called a footprint of the living individual.

Alternatively preferably, the baseline entropy production is estimated on the basis of respective data calculated and collected for a representative population of the kind of living individuals. In this case, individual characteristics of the particular living individual who is being monitored (such as body-mass-index (BMI) and age) may be further taken into account in the calculation of the stress entropic load. Although such an approach is less "tailored" for a particular individual to be monitored and is thus less precise, it represents a suitable alternative in cases when there is no time to determine the baseline entropy production for this particular individual in advance.

Also preferably, the baseline entropy production is simply assumed to have a constant rate, i.e. the baseline entropy production is a linear function of time duration. The constant rate may then be estimated on the basis of available data of a representative population or on some available data about the particular individual.

Further features and advantages of the present invention are set forth in dependent claims.

## BRIEF DESCRIPTION OF THE DRAWINGS

Additional features and advantages of the present invention will become apparent in the following and more particular description as illustrated in the accompanying drawings, wherein:

Fig. 1     is a conceptual-level block diagram of a system that may be provided in accordance with some embodiments;

Fig. 2     graphically illustrates the overall workflow of stress monitoring within the "digital twin" system;

Fig. 3     represents the input into the system;

Fig. 4     is a graphic representation of the data gathering and normalization process;

Fig. 5     provides a detailed illustration of the SEL calculation process;

Fig. 6     illustrates standardized scenarios for baseline delta SEL gathering;

Fig. 7     provides a detailed illustration of the baseline creation process for a twinned individual;

Fig. 8     provides a detailed illustration of the delta processing;

Fig. 9     is a graphic representation of the footprinting process;

Fig. 10     is a graphic representation of the evaluation process including a comparison with the footprint data;

Fig. 11     is a graphic representation of the evaluation process including a comparison with the library data;

Fig. 12     is a graphic representation of alerts, prognostic and simulations;

Fig. 13     provides an exemplary graph of rates of $O_2$ consumption and $CO_2$ liberation for a simulated human;

Fig. 14     provides an exemplary illustration of change of body temperatures of the simulated human over time;

Fig. 15     provides examples of characteristics of the outer environment of the living individual (air);

Fig. 16     illustrates the entropy production rate (overall and basal) as a function of time on the basis of the simulated values shown in Figs. 12 to 14;

Fig. 17     is an illustration of the cumulated entropy production (overall and basal) as a function of time on the basis of the simulated values shown in Figs. 12 to 14;

Fig. 18     shows the SEL rate as a function of time for the example of the foregoing figures; and

Fig. 19     illustrates the calculated stress entropic load as well as a unique function thereof representing the relative risk of failure, to be compared with a threshold for estimating the time of adaptation failure of the individual.

## DETAILED DESCRIPTION

The concept of a digital twin has recently emerged in industry, where it is often associated with the concomitant use of twinned physical systems. The use of a digital image of a machine and/or living systems is potentially extremely useful, but the construction of a twinned physical system is currently impossible for living systems at a given level of detail. Therefore, the development of novel approaches not requiring the existence of physical twins is imperative. In this application, we present a digital twin of a living system based completely on thermodynamic phenomena that occur at the interface between the living system and the surrounding environment that make it possible to predict the future development (e.g. stability) and/or other parameters of the living system without the existence of the physical twin. The main advantage of this approach lies in a combination of holistic thermodynamic approaches and learning algorithms that make it possible to constantly re-adjust the model.

According to some embodiments, an apparatus implements a digital twin of a twinned person in such a way that one or more sensors measure chosen parameters of the twinned individual and a computer processor receives data from the sensors. Based on the received data, the current level of stress is monitored and quantified, while an auto-iterative mechanism of digital twin simulation allows for the assessment of stress severity in a given individual. For example, values measured by sensors may show that the stress entropic load (SEL) of a particular individual is increasing faster than normal, which signalizes a severe level of stress.

Some embodiments comprise: means for sensing, by one or more sensors, several selected parameters of a twinned individual, for example, those given in Table 1 (see further at Eq. (5)) and Table 2

The following disclosure describes the present invention according to several embodiments directed at methods, systems, and apparatuses related to using digital twins (DTs) estimation of stress in humans. Employing DTs provides information relevant to stress level estimation from the underlying data and simulation model. This allows end users to monitor stress levels by having access to detailed information regarding past, current and predicted stress production based on thermodynamic approaches. Such information cannot be provided by the traditional stress estimation methods where vital signs are monitored (e.g., blood pressure, heart rate). When abnormalities in vital signs are detected, the stress levels can be difficult to interpret as other processes can also substantially alter vital signs dynamics (such as a physical activity that does not necessarily have to constitute stress). Also, using a DT architecture, stress levels can be checked automatically or manually on a much more frequent basis, which, in turn, results in quicker fault detection. The detailed information provided by each DT can form the basis for simulations which can be used to develop predictive safety time estimation procedures based on patterns of an individualized stress generation in real-life scenarios.

Embodiments of the present invention address and overcome one or more of the above shortcomings and drawbacks of conventional stress measurement methods by providing methods, systems and apparatuses related to the creation and use of digital twins (DT) for stress estimation in humans and animals. The DT technology described herein may be used, for example, to provide physicians with dynamic, structured data regarding stress levels using historical data, real-time data as well as full data repositories and simulation models. Using the DT paradigm, physicians, managers or even military personnel might interact with each other for a better quality of response to an individual's stress and for more effective therapeutic/intervention results.

In many situations, it would be useful to make an assessment and/or predictions about the adaptation of a living system, e.g., humans. For example, it may be helpful to predict the safety time (time to adaptation failure) in specific scenarios, such as a challenge to noxious agents or to help plan when the living system should be withdrawn before it becomes irreversibly damaged. It is conceivable that in some cases, an expected safety time of a living system may be estimated from probabilities of failure of the system's subsystems, such as organ systems (for example, of the cardiovascular system) too. Such an approach, however, might tend to be more reactive than proactive or predictive.

Thanks to a great advancement in the field of technical design of sensors, communication pathways as well as data integration and modeling, it is now possible to monitor and continuously assess multiple parameters of a living system. Therefore, it seems logical to use these modern tools to monitor and predict the level of perceived/objectively produced stress and consequently to estimate the safety time of a living system in given conditions. This would be a significant advance for prognostics in theoretical physiology as well as in a clinical setting, and discovering a system and methodology to do so accurately and efficiently would help reduce adaptation failures of living systems that may present for example, as a multi-organ failure or failure of single organ systems (e.g., cardiovascular collapse). According to some embodiments described herein, this information may be provided by a "digital twin" of a twinned living system.

The digital twin is a concept of making a replica of a real living or non-living system in a digital world in the form of a numerical representation. Here we describe a digital twin representing the production of stress-associated entropy within a living system that is indicative of the state of the living system. In order to generate the digital twin, the systems and methods obtain status data from multiple different sensors attached to the twinned individual. For example, the status data may indicate a state of health or disease of the living system. The status data obtained from different sensors may differ in one or more sampling rates or resolutions for at least two of the different sensors. For example, the status data obtained from a first sensor may be at some frequency and some sampling rate and the status data obtained from the second sensor may be at a second different frequency and a second different sampling rate. In such a case, systems and methods modify at least some of the status data with different sampling rates or resolutions so that the modified status data has a common sampling rate or a common sampling resolution. This processing may be called "data normalization". The systems and methods generate a digital twin matrix. It is noted that the term "matrix" is used in this disclosure in its general meaning as "the set of conditions that provides a system in which something grows or develops" (Cambridge Dictionary online, https://dictionary.cambridge.org/de/worterbuch/englisch/matrix). This does not necessarily imply the mathematical form of a matrix. The digital twin matrix is indicative of the state of the living system and that makes it possible to determine the status characteristics of the living system or simulate functioning of the living system. The digital twin matrix can, e.g., form a phenomenological linear dynamic model for the living system that may relate to one or more temperature values (for example, ambient temperature, core temperature or surface temperature of the twinned subject) as well as other parameters such as the molar amounts of inhaled oxygen and exhaled carbon dioxide.

A digital twin may be used to estimate the safety time of a twinned living system using sensors, communications, modeling, history, data library, and computation. It may do so in a useful time frame, that is, meaningfully before a projected occurrence of adaptation failure or suboptimal performance of a living system. It might comprise a code object with parameters and dimensions of its twinned living system and dimensions that provide measured values, and it keeps the values of those parameters and dimensions current by receiving and updating values via outputs from sensors attached on the living system. The digital twin may, according to some embodiments, be upgraded after the occurrence of unexpected events and novel additional data, such as the discovery and identification of exogenous variables, which may improve accuracy. The digital twin may also be used to evaluate several twinned individuals and choose the most suitable candidate for a planned high-stress mission. The digital twin may comprise a real-time efficiency as well as the life consumption state estimation device. It may contain a specific or "per organism" portfolio of system models and organism-specific sensors. It may receive data and track a single specific individual over its lifetime with observed data and calculated state changes. Some digital twin models may include a mathematical or functional form that is the same for like organisms, but it has tracked parameters and state variables that are individualized.

The digital twin may be attached to the twinned living system and run autonomously or globally with a connection to external resources using the Internet of Things (IoT) or other data services. The instantiation of the digital twin's software may take place at several locations. A digital twin's software could reside near the living system (for example, within a wearable) and be used to help monitor the state of the living system. Another location might be at a distant headquarters level where system-level digital twin models may be used to help determine optimal operating conditions for the desired outcome, such as maximization of performance of individual living systems (e.g., humans) or their groups. In addition, a digital twin's software could reside in the cloud, implemented on a server remote from the wearable. One advantage of cloud location might be that it allows including scalable computing resources to solve computationally intensive calculations required to converge a digital twin model producing an output vector y.

It should be noted that multiple but different digital twin models for a specific living system, such as an individual human, could reside at all possible locations. Each location might, for example, be able to gather different data, which may allow for better observation of the living system states and hence determination of the safety parameters, especially when the different digital twin models exchange information.

A Per Organism digital twin may comprise a mathematical representation or a mathematical model that describes the current state of the individual as well as the current state of the surrounding environment. This is often done using a mathematical framework based on thermodynamic models of stress. The basic variable used in the model may be the stress entropic load (SEL) that represents the amount of entropy generated within the living system due to adaptation. Such a digital twin may be configured to function as a continually tuned digital twin and/or adaptable digital twin. A continually tuned digital twin is continually updated as its twinned living system is on-operation. An adaptable digital twin is designed to adapt to new scenarios and new system configurations. Furthermore, an adaptable digital twin may be transferred to another system or class of systems, and/or one of a plurality of interacting digital twins that are scalable over a population and may be broadened to not only model an individual living system but also to provide predictions of their interactions and outcomes of such interactions.

The systems and methods determine the stress status of the living system based on the digital twin matrix of the living system. For example, a low-stress score of the living system may indicate that the living system is capable of staying within given conditions for a prolonged length of time (safety time). Alternatively, a high-stress score of the living system may indicate that the living system will not be capable of adapting within given conditions (prediction of breakdown or disease) to perform the impending task. Optionally the systems and methods may predict a likelihood of adaptation failure of the living system based on the stress score of the living system. For example, a low-stress score may indicate that the living system will not likely fail under a particular set of circumstances (for example, psychosocial stress load, physical exhaustion). Alternatively, a high-stress score may indicate that the living system will likely fail under a particular set of circumstances. Optionally, the systems and methods simulate the operation of the living system based on the digital twin matrix. The simulation can be based on a variety of environmental or intrinsic factors representative of an environment in which the living system may develop stress. For example, environmental factors may include temperature, humidity, wind, precipitation or the like, or intrinsic factors may include genetic background, epigenetic background, physiological parameters, behavior or the like. Optionally, the systems and methods may predict a likelihood of adaptation failure of the living system based on the simulated operation. For example, the simulated operation may indicate whether the living system will likely fail or not fail under a simulated set of circumstances (for example, environmental factors including psychosocial load, etc.). The systems and methods are unable to change or control the actual operation of the living system based on one or more of the stress scores of the living system or the simulated operation of the living system. However, a simulated operation of the living system may indicate that the living system will likely fail when subjected to the simulated factors. Therefore, based on the simulation outcomes, the device will trigger implementation of specific countermeasures in order to prevent system failure.

At least one technical effect of the subject matter described herein is that systems and methods can be used to prevent failure of adaptation of the living system in given environmental conditions by utilizing the digital twin matrix to determine the stress score and simulate the operation of the living system. This can result in reduced safety time in given conditions (for example, relative to not determining a stress score or simulating the operation of the living system). At least one technical effect isolates poorly adapting living systems. At least one technical effect simulates the performance of a given living system in given conditions. At least one technical effect predicts a failure of adaptation of the living system in given conditions or predicts a failure of another living system. This can result in reduced safety/health/economic costs.

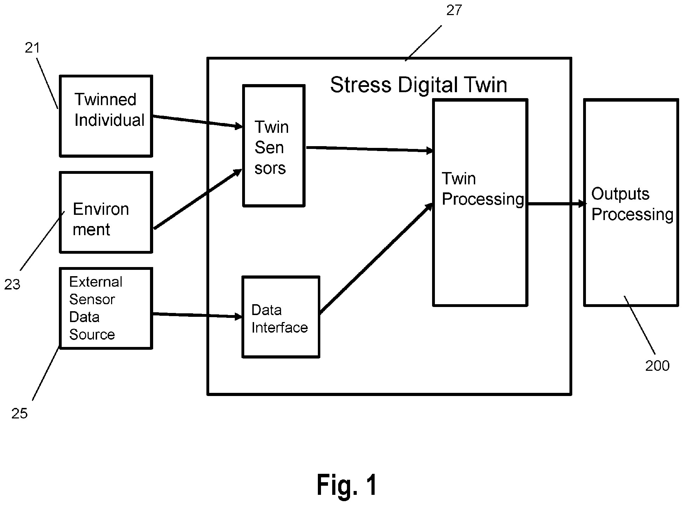

In the following, details of the system of the present invention and the processing thereof will be described with reference to Figs. 1 to 12.

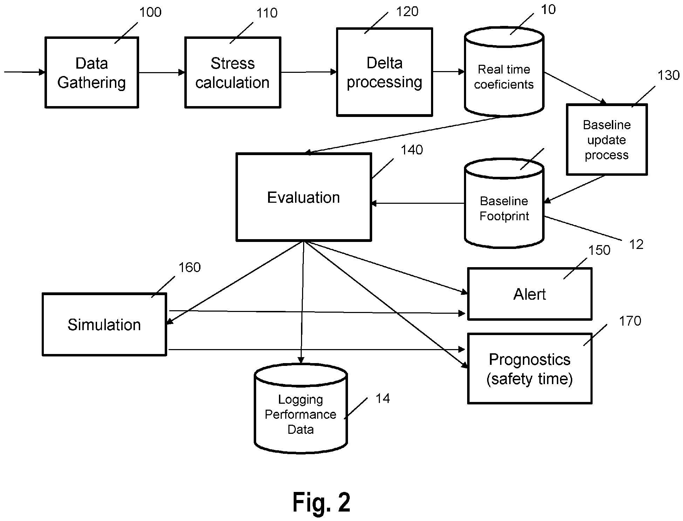

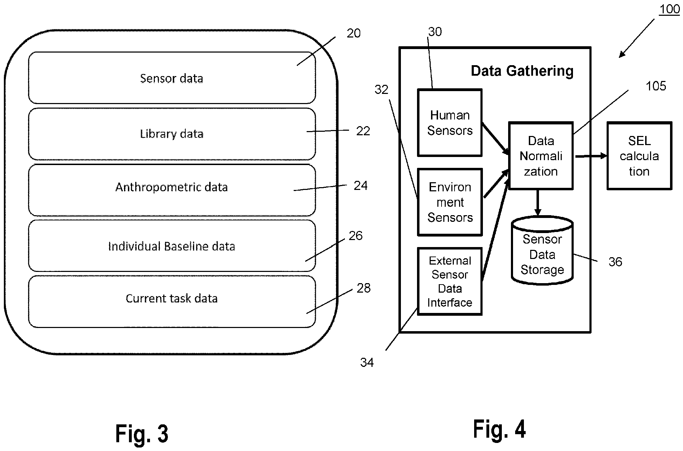

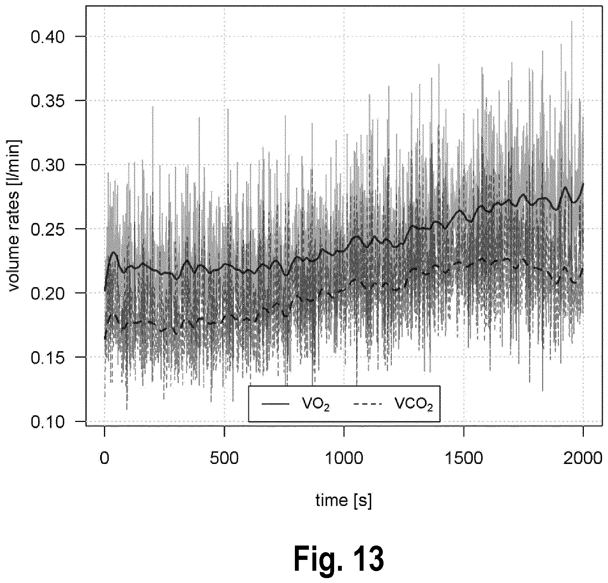

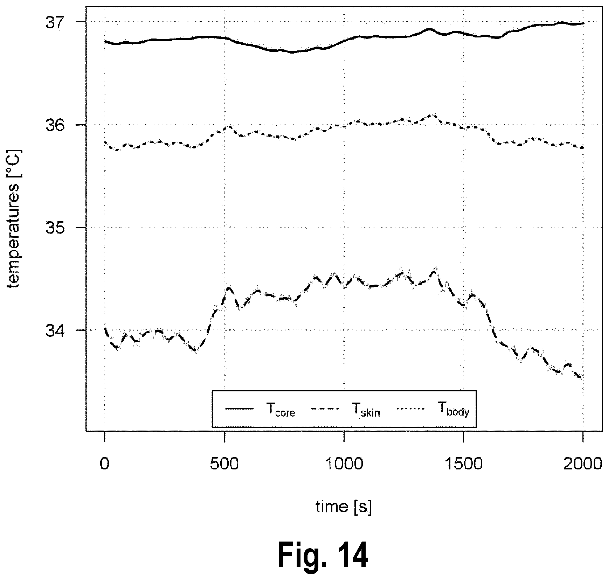

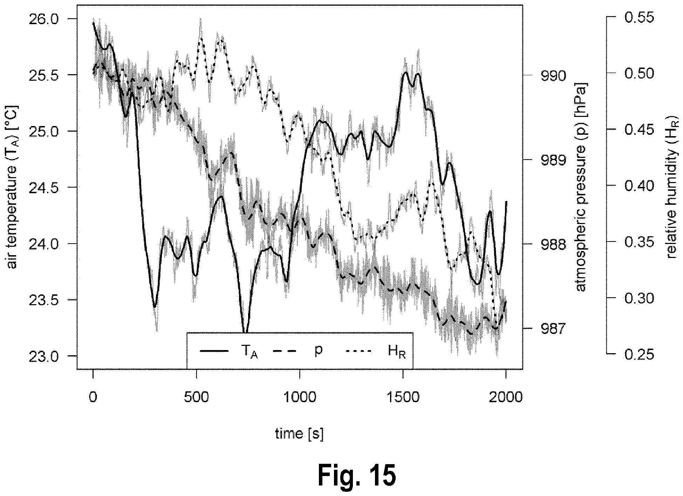

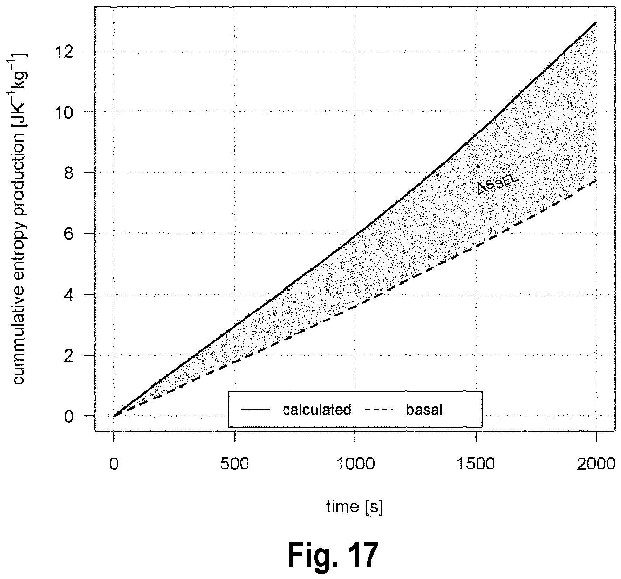

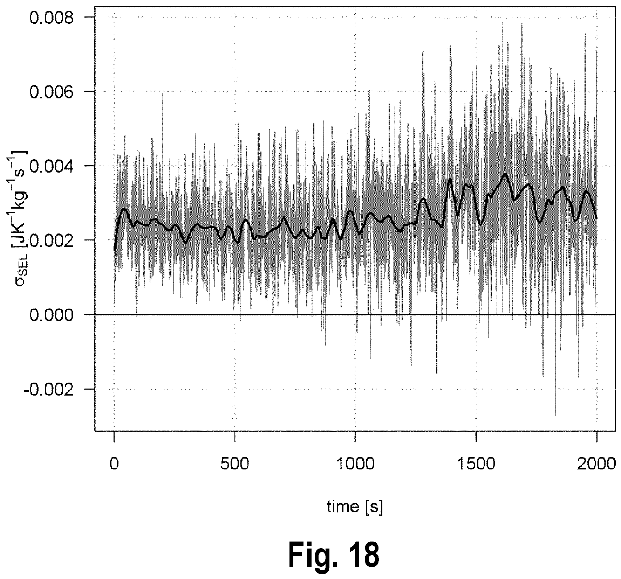

While Figures 1 and 2 show the overall concept of the system, Figures 3 to 12 describe its particular segments. In the following text, a more detailed description of the general processes within the system is provided. Moreover, an example using the simulated data will be further explained with reference to Figs. 13 to 19.

Fig. 1 is a conceptual-level block diagram of a system that may be provided in accordance with some embodiments.

The central item in Fig. 1 is the "Stress Digital Twin" 27, i.e. the system for monitoring a stress level of a real living individual. The basic processing by the stress digital twin 27 is summarized as "Twin Processing" in the block on the right-hand side within block 27. An overall workflow of twin processing will be described below with reference to Fig.

2. The real living individual is labeled "Twinned Individual" 21 in Fig. 1.

Twin processing is performed, on the one hand, on the basis of sensor data obtained by a plurality of sensors. The sensors include, in particular, human sensors that measure data directly on the body of the twinned individual 21. On the other hand, there are optionally also environment sensors for measuring data in the environment 23 of the twinned individual. Further, additional data from external data sources 25 can be taken into account. A data interface is foreseen for receiving such data to be processed.

On the right-hand side of the drawing, a block representing the output processing 200 is shown.

The workflow of Fig. 2 representing the actual twin processing starts with data gathering 100. This includes, in particular, gathering data which is directly measured on the living individual, with the help of respective sensors. In addition, there may be data obtained by means of environment sensors. Particular examples will be given below.

Stress calculation may also include variables other than those measured by human and environment sensors. An example of such additional variables is a clothing coefficient which may be crucial for proper interpretation of some values measured by human sensors (for example, the same temperature under different cloths may be a result of different heat production and thus different SEL).

Subsequently, thermodynamic calculations for obtaining an overall entropy production on the basis of the gathered data are performed in block 110. Specific examples of performing this process will be further detailed below. The actual stress entropic load is obtained by subtracting an amount of entropy production corresponding to a stress-free situation that has been obtained in advance (baseline entropy production) from the calculated overall entropy production.

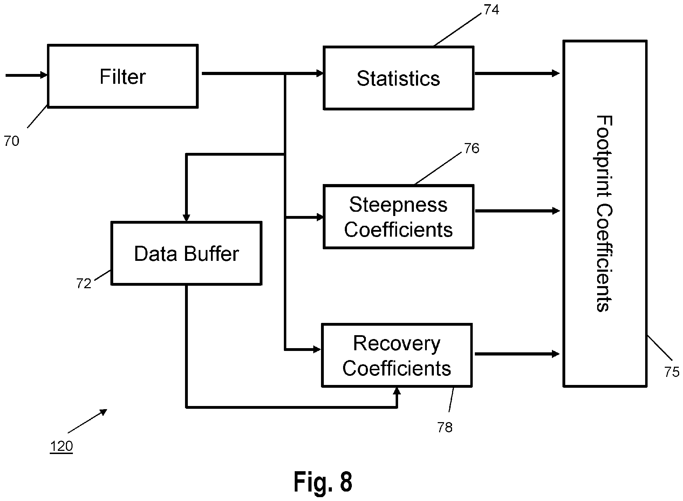

In subsequent block 120, an algorithmic evaluation of SEL changes in time is performed. This processing step is called "Delta processing" and will be detailed below with reference to Fig. 8.

As a result, real-time data 10 of stress entropic load are obtained and stored in a database.

As will be further described, under the condition of respective stress-free environmental conditions, the same processing which has been described with reference to the blocks in the first line of Fig. 2 can also be used for calculating real-time baseline entropy production data. In that case, there is, of course, no subtraction. In this manner, a baseline footprint 12 can be obtained and regularly updated 130 by repeating foregoing steps 100, 110, and 120 over time in an iterative manner.

Digital twin processing thus utilizes the same input cascade processing as a baseline creation process. In real time, it processes all gathered sensor data, calculates SEL values using the stress calculation process, and produces statistical information and coefficients using the delta processing component 120.

Subsequently, the actual evaluation 140 is performed on the basis of the thus generated real-time data 10 and the baseline footprint 12.

Evaluation process 140 is the core of the digital twin principle allowing real-time data of a living system to be compared with its numerical representation using either the baseline footprint of a living system or using library model approximation. Further details will be given below.

As a result of the data evaluation 140, performance data of the living individual are obtained and locked in a respective database 14. Based thereon, a respective indication can be output via the output interface (not shown in Fig. 2). This includes, in particular, an alert 150, in case any danger such as failure of the monitored living individual threatens. By means of extrapolation and/or simulation, prognostics 170 can be further made, and a respective output, including alerts 150, can be made based thereon. This further includes the possibility of making a simulation 160 showing the expected behavior of the monitored living individual in the future as well, on the basis of the available real-time data (as well as the baseline footprint in some embodiments). In the context of the present disclosure, extrapolation refers basically to the use of individual data for prediction of the behavior of the variable later in time. It does not take into account any amendments to initial conditions, just the starting values in the individual. Simulation, on the other hand, refers to more complex process using amendments of the initial as well as later conditions to alter the outcome.

Fig. 3 illustrates the basic inputs in the one exemplary embodiment of the system.

The system utilizes a whole range of inputs, including, sensor data 20, library data 22, anthropometric data 24 of the subject and individual baseline data 26 as well as current task data 28.

As already mentioned, sensor data 20 include at least data obtained by sensors for measuring parameters directly on the body, as well as optionally data obtained by environment sensors.

Library data 22 may include data obtained in advance characterizing an average population of individuals of the respective kind, for instance, humans or humans of a predetermined age range etc.

Fig. 4 is a graphic representation of the data gathering and normalization process

Fig. 4 provides a detailed description of the process of data gathering 100 necessary for the calculation of stress levels that is further described in the frame of Fig 4. The described process requires two basic groups of sensors. The first group of sensors, human sensors 30, sense values of one or more variables from the measured individual (e.g., the temperature of a certain body part). The second group of sensors, environment sensors 32, sense one or more variables from the environment (e.g., atmospheric pressure). Data from both groups can be obtained from a sensor device attached to a digital twin or may utilize an external source 25 of data gathered over a communication interface 34. A data normalization process 105 may be used to pre-process input sensor data for different sensor types or data from external interface, for example unit conversion, resolution adjustment, etc., and filtering out artifacts (misleading or confusing alteration in data resulting from flaws in technique or equipment). A sensor data storage 36 may be provided to store normalized sensor data (or, alternatively, raw sensor data, not shown in Fig. 4).

Values measured by both groups of sensors are then continuously used to calculate stress entropic load (SEL) for a measured individual. This processing, indicated in the respective block on the right hand side of Fig. 4, basically corresponds to the processing in steps 110 and 120 of Fig. 2. It is important to note that the order of sensor groups is arbitrary and that in some applications, the second group of sensors. i.e., environment sensors, may not be necessary for an accurate calculation of SEL.

Stress calculation, i.e., calculation of SEL illustrated in Fig. 4, may also include variables other than those measured by Human and Environment sensors. An example of such additional variables is the aforementioned clothing coefficient.

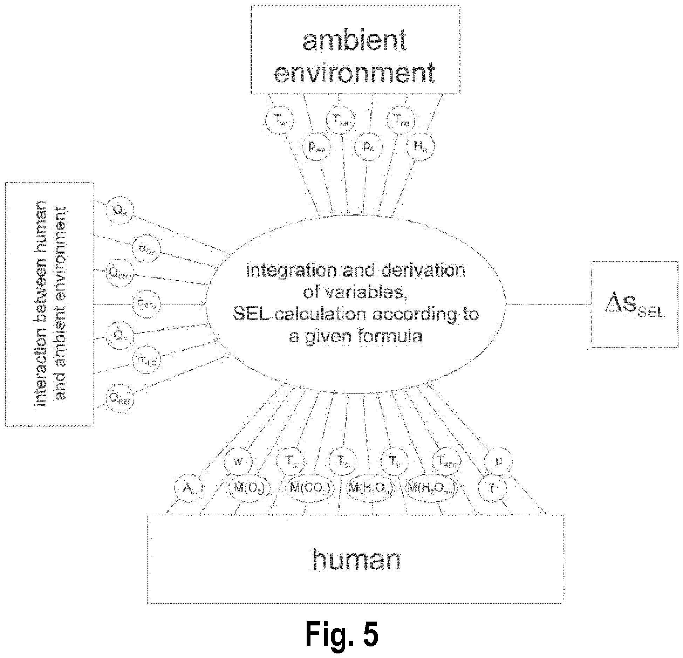

Fig. 5 illustrates a detailed description of the calculation process of stress based on the thermodynamic processes.

Fig. 5 illustrates the variables included in the calculation of the stress entropic load (SEL), which mirrors current levels of stress. A detailed description of the calculation is provided below.

The calculation model relies on two prerequisites. Firstly, living organisms are open systems and their overall changes in the entropy can be expressed as: $dS_{TOT} = dS_{PROD} + dS_{FLOW}$ where $dS_{FLOW}$ denotes changes in entropy corresponding to the flow of energy and mass across system boundaries and where $dS_{PROD}$ denotes changes in entropy corresponding to processes taking place inside the system (entropy production). The second prerequisite is the universality of the second law of thermodynamics, which states that $dS_{PROD} \geq 0$.

Based on these universally accepted concepts, it is further presumed that $dS_{PROD}$ includes a base component ($dS_{BASAL}$) and reactive component ($dS_{SEL}$). In other words, $dS_{PROD} = dS_{BASAL} + dS_{SEL}$. $dS_{BASAL}$ corresponds to the intrinsic increase of system complexity independent of its surroundings associated with growth, development, and aging. $dS_{SEL}$ corresponds to an increase in entropy due to a wide range of environmental influences which reflects the energy cost of adaptation, i.e. what is generally to be understood as "stress" in the present disclosure.

To make individual rates of change of system entropy comparable, it is necessary to introduce the concept of specific rate of change of system entropy per unit mass ($\sigma(t)$), i.e. presume that the entropy production is mass-dependent:

$$\sigma(t) = \frac{\dot{S}(t)}{m(t)}.$$

Stress entropic load ($s_{SEL}$) is then acquired by integrating $\sigma_{SEL}$ and is defined as

$$s_{SEL} := \int_{t_{conc}}^{t} \sigma_{SEL}(\tau)d\tau \ (1),$$

where SEL is defined as an integral from the time of conception $t_{conc.}$ of a given individual to a later point in time of the specific rate of change of the entropy $\sigma_{SEL}$ of the individual. Here and in the following, lowercase letters "s" indicate mass specific variables, i.e. $s = S/m$.

The above is the key theoretical expression of the entropy-based stress model according to the present invention.

The theoretical expression established above allows deriving a change (increase) in SEL for a reasonable and practically applicable time interval $[t_1, t_2]$, calculating it as the difference between stress entropic load values at the extreme points of the interval:

$$\Delta_{[t_1; t_2]} s_{SEL} := s_{SEL}(t_2) - s_{SEL}(t_1) = \int_{t_1}^{t_2} \sigma_{SEL}(\tau)d\tau \ (2)$$

where $t_2 \geq t_1 \geq t_{conc.}$

In case the given interval is clearly delineated, the following abbreviated equation may be employed: $\Delta S_{SEL} = s_{SEL}(t_2) - s_{SEL}(t_1)$ Within a given interval $[t_1, t_2]$, SEL change in produced entropy is calculated as the difference between the overall change of entropy and change of entropy due to mass and energy flows across system boundaries (all values per unit of mass):

$$\Delta s_{PROD} = \Delta s_{TOT} - \Delta s_{FLOW} \ (3)$$

where the following increases - $\Delta s_{TOT}$, $\Delta s_{FLOW}$ and $\Delta s_{BASAL}$ - are defined analogously to $\Delta s_{PROD}$. SEL has been defined as part of entropy production, i.e. the difference between total entropy production and entropy production associated with basal metabolic function. It therefore follows that

$$\Delta s_{SEL} = \Delta s_{PROD} - \Delta s_{BASAL} \quad (4)$$

As long as the system is not subject to stress (i.e. in case $\Delta s_{SEL} = 0$), then $\Delta s_{BASAL} = \Delta s_{PROD}$, i.e. an increase in entropy caused by basal metabolic function is directly equal to an increase in entropy production.

The above may be summed up as

$$\Delta s_{SEL} = \Delta s_{PROD} - \Delta s_{BASAL} = \Delta s_{PROD(stressed)} - \Delta s_{PROD(no\ stress)} =$$

$$= [\Delta s_{TOT} - \Delta s_{FLOW}]_{(stressed)} - [\Delta s_{TOT} - \Delta s_{FLOW}]_{(no\ stress)}$$

Combining the above equations facilitates the calculation of the stress entropic load change of an individual during the time interval *[0,t]* in the most general form, expressed as

$$\Delta s_{SEL}(t) = \int_0^t \frac{1}{w(s)} \left[ \frac{\dot{Q}_{prod}(s)}{T_B(s)} \left( 1 - \frac{\dot{T}_B(s)}{T_B(s)} \right) + \dot{Q}_{R,out}(s) \left( \frac{1}{T_S(s)} - \frac{1}{T_B(s)} \right) - \dot{Q}_{R,in}(s) \left( \frac{1}{T_{MR}(s)} - \frac{1}{T_B(s)} \right) + \right.$$

$$+ \dot{Q}_{CNV}(s) \left( \frac{1}{T_S(s)} - \frac{1}{T_B(s)} \right) + \dot{Q}_E(s) \left( \frac{1}{T_C(s)} - \frac{1}{T_B(s)} \right) + \dot{Q}_{RES}(s) \left( \frac{1}{T_{RES}(s)} - \frac{1}{T_B(s)} \right) +$$

$$+ \frac{\dot{T}_B(s)}{T_B(s)^2} \left( Q_{R,out}(s) - Q_{R,in}(s) \right) + Q_{CNV}(s) \left( \frac{\dot{T}_B(s)}{T_B(s)^2} - \frac{\dot{T}_S(s)}{T_S(s)^2} \right) + Q_E(s) \left( \frac{\dot{T}_B(s)}{T_B(s)^2} - \frac{\dot{T}_C(s)}{T_C(s)^2} \right) +$$

$$+ Q_{RES}(s) \left( \frac{\dot{T}_B(s)}{T_B(s)^2} - \frac{\dot{T}_{RES}(s)}{T_{RES}(s)^2} \right) -$$

$$- \sigma_{O_2}(s) \dot{M}(O_2)(s) + \sigma_{CO_2}(s) \dot{M}(CO_2)(s) - \sigma_{H_2O}(s) \dot{M}(H_2O_{in})(s) + \sigma_{H_2O}(s) \dot{M}(H_2O_{out})(s) -$$

$$- \dot{\sigma}_{O_2}(s) M(O_2)(s) + \dot{\sigma}_{CO_2}(s) M(CO_2)(s) - \dot{\sigma}_{H_2O}(s) M(H_2O_{in})(s) + \dot{\sigma}_{H_2O}(s) M(H_2O_{out})(s) \Big] ds$$

$$- \int_0^t \sigma_{prod,no\ stress}(s) ds \qquad (5)$$

As can be seen from Table 1 below, typical parameters on which the evaluation of the stress level of a twinned living individual is based may include each one or any combination of temperature values of predetermined body parts as well as their change rates, heat exchange, production and absorption rates as well as the respective total heat amounts, exchange rates of oxygen, carbon dioxide and water as well as the respective amounts of the exchanged gases, without being limited to these parameters.

Table 1

| | | | |
|---|---|---|---|
| $\omega$ | ... | weight of a body | [kg] |
| $T_B$ | ... | average body temperature | [K] |
| $T_S$ | ... | average skin temperature | [K] |
| $T_C$ | ... | core temperature | [K] |
| $T_{RES}$ | ... | temperature of expired air | [K] |
| $T_{MR}$ | ... | mean radiant temperature | [K] |
| $\dot{T}_B$ | ... | rate of average body temperature | [Ks$^{-1}$] |
| $\dot{T}_S$ | ... | rate of skin temperature | [Ks$^{-1}$] |
| $\dot{T}_C$ | ... | rate of core temperature | [Ks$^{-1}$] |
| $\dot{T}_{RES}$ | ... | rate of temperature of expired air | [Ks$^{-1}$] |
| $Q_{prod}$ | ... | total produced heat | [J] |

(continued)

| | | | |
|---|---|---|---|
| $Q_{R,in}$ | ... | total amount of heat absosbed by radiation | [J] |
| $Q_{R,out}$ | ... | total amount of beat eliminated by radiation | [J] |
| $Q_{CNV}$ | ... | total amount of beat lost by convection | [J] |
| $Q_E$ | ... | total amount of beat lost by evaporation | [J] |
| $Q_{RES}$ | ... | total amount of beat lost by respiration | [J] |
| $\dot{Q}_{prod}$ | ... | heat production rate | [W] |
| $\dot{Q}_{R,in}$ | ... | rate of absorbed radiative heat | [W] |
| $\dot{Q}_{R,out}$ | ... | rate of eliminated radiative heat | [W] |
| $\dot{Q}_{CNV}$ | ... | convective heat loss rate | [W] |
| $\dot{Q}_E$ | ... | evaporative heat loss rate | [W] |
| $\dot{Q}_{RES}$ | ... | respirative heat loss rate | [W] |
| $\sigma_{O_2}$ | ... | entropic content of $O_2$ | $JK^{-1}l^{-1}$ |
| $\sigma_{CO_2}$ | ... | entropic content of $CO_2$ | $JK^{-1}l^{-1}$ |
| $\sigma_{H_2O}$ | ... | entropic content of $H_2O$ | $JK^{-1}l^{-1}$ |
| $\dot{\sigma}_{O_2}$ | ... | rate of entropic content of $O_2$ | $JK^{-1}l^{-1}s^{-1}$ |
| $\dot{\sigma}_{CO_2}$ | ... | rate of entropic content of $CO_2$ | $JK^{-1}l^{-1}s^{-1}$ |
| $\dot{\sigma}_{H_2O}$ | ... | rate of entropic content of $H_2O$ | $JK^{-1}l^{-1}s^{-1}$ |
| $M(O_2)$ | ... | amount of $O_2$ uptake | [l] |
| $M(CO_2)$ | ... | amount of $CO_2$ liberation | [l] |
| $M(H_2O_{in})$ | ... | amount of $H_2O$ uptake | [l] |
| $M(H_2O_{out})$ | ... | amount of $H_2O$ liberation | [l] |
| $\dot{M}(O_2)$ | ... | $O_2$ uptake rate | $[ls^{-1}]$ |
| $\dot{M}(CO_2)$ | ... | $CO_2$ liberation rate | $[ls^{-1}]$ |
| $\dot{M}(H_2O_{in})$ | ... | $H_2O$ uptake rate | $[ls^{-1}]$ |
| $\dot{M}(H_2O_{out})$ | ... | $H_2O$ liberation rate | $[ls^{-1}]$ |
| $\sigma_{prod.\ no\ stress}$ | ... | specific entropy production rate of a body in "unstressed" state | $JK^{-1}kg^{-1}s^{-1}$ |

As will be further detailed below, the entropy production rate of the individual in an unstressed state will normally be obtained and stored in advance by performing respective real-time measurements and calculations according to equation (5) - without calculation and subtracting the second integral - under conditions of no stress. This corresponds to obtaining and storing the baseline footprint 12 in Fig. 2. In other words, an individual 21 whose stress level is to be monitored undergoes a kind of "calibration process" in advance, in order to obtain an initial baseline footprint 12. As has already been indicated, this baseline footprint may be also updated by repeating the processing under stress-free conditions. The entropy production rate under stress-free conditions is also called "Resting Metabolic Rate" (RMR).

However, alternatively, there is another way of obtaining a suitable estimation of a baseline footprint without having to perform the calibration procedure for a particular individual 21 to be monitored, for instance, when there is insufficient time. Namely, when baseline data have been measured, processed and collected, it is possible to estimate a particular individual's baseline entropy production based on individual characteristics such as BMI, age and/or other characteristics.

The variables necessary for calculating SEL are typically time-dependent. However, these variables may also be calculated from other measurable variables based on known formulas. Specifically, heat exchanges can be expressed as follows, with all variables appearing listed in Table 2.

$$\dot{Q}_{prod}(t) = M(t) \qquad\qquad [W]$$

$$\dot{Q}_R(t) = \dot{Q}_{R,out}(t) - \dot{Q}_{R,in}(t) = \varepsilon A_e \sigma_{SB} T_S^4 - A_e \eta \sigma_{SB} T_{MR}^4 \qquad\qquad [W]$$

$$\dot{Q}_{CNV}(t) = h_c A_e (T_S - T_A) = 1.87 A_e \sqrt{\frac{p_{atm}}{p_0}} (T_S - T_A)^{\frac{5}{4}} \dot{Q}_E(t) = \dot{Q}_D(t) + \dot{Q}_{SW}(t) \qquad \text{[W]}$$

$$\dot{Q}_D(t) = A_e \lambda \mu (p_S - p_A) = A_e \lambda \mu [p(T_S) - H_R p(T_A)] \qquad \text{[W]}$$

$$\dot{Q}_{SW}(t) = \left[ (w_{in} - w_f) - (f + u) - 0.019 \overline{M(O_2)}(44 - p_A)t \right] \frac{l}{60t} \qquad \text{[W]}$$

$$\dot{Q}_{RES}(t) = A_e [0.0014 M(T_{EX} - T_{DB}) + 0.0017 M(58.7 - p_A)] \qquad \text{[W]}$$

$$p(T) = 611.21 exp \left[ \left( 18.678 - \frac{T - 273.15}{234.5} \right) \frac{T - 273.15}{T - 16.01} \right] \qquad \text{[Pa]}$$

Table 2

| | | |
|---|---|---|
| $\varepsilon$ | emissivity of human skin | [-] |
| $A_e$ | effective area of human skin | [m$^2$] |
| $\sigma_{SB}$ | Stefan-Boltzmann constant (5.670367·10$^{-8}$ Wm$^{-2}$K$^{-4}$) | |
| $T_S$ | average skin temperature | [K] |
| $\eta$ | absorbity of human skin | [-] |
| $T_{MR}$ | mean radiant temperature | [K] |
| $h_c$ | convective heat loss coefficient | [Wm$^{-2}$K$^{-1}$] |
| $T_A$ | ambient air temperature | [K] |
| $P_{atm}$ | atmospheric pressure | [Pa] |
| $p_0$ | atmospheric pressure at standard conditions (101 325 Pa) | |
| $\lambda$ | latent heat of evaporation of sweat (2 430 Jg$^{-1}$) | |
| $\mu$ | permeance coefficient of the skin (1.270608·10$^{-6}$ gs$^{-1}$m$^{-2}$Pa$^{-1}$) | |
| $p_S$ | saturated air vapor pressure at skin temperature | [Pa] |
| $p_A$ | saturated air vapor pressure at ambient air temperature | [Pa] |
| $p(T)$ | saturated air vapor pressure at temperature $T$ at atmospheric pressure 1 atm | [Pa] |
| $H_R$ | relative humidity in the air | [-] |
| $W_{in}$ | initial weight | [g] |
| $W_f$ | final weight | [g] |
| $f$ | fluid/food intake | [g] |
| $u$ | urine/feces loss | [g] |
| $M(O_2)$ | average oxygen uptake rate during measurement | [ls$^{-1}$] |
| $t$ | length of measurement | [s] |
| $M$ | metabolic heat production | [W] |
| $T_{DB}$ | dry bulb temperature | [K] |

Including the assumptions that have been made above in the expression (5) for calculating the stress entropic load change $\Delta s_{SEL}(t)$ provides the following derived formula for the calculation of SEL (Eq. 6):
Using above mentioned formulas for calculating different kind of heats and assuming several approximations, one can arrive at the following form of SEL function:

$$
\begin{aligned}
\Delta s_{SEL}(t) = \frac{1}{w}\int_0^t \Bigg\{ & \frac{M(s)}{T_B(s)}\left(1 - \frac{\dot{T}_B(s)}{T_B(s)}\right) + \varepsilon A_e \sigma_{SB} T_S(s)^4 \left(\frac{1}{T_S(s)} - \frac{1}{T_B(s)}\right) - \varepsilon A_e \sigma_{SB} T_S(s)^4 \left(\frac{1}{T_S(s)} - \frac{1}{T_B(s)}\right) + \\
& + 1.87 A_e \sqrt{\frac{p(s)}{p_0}}\left(T_S(s) - T_A(s)\right)^{\frac{5}{4}}\left(\frac{1}{T_S(s)} - \frac{1}{T_B(s)}\right) + \\
& + A_e \lambda \mu \left[ 611.21 exp\left(\left(18.678 - \frac{T_S(s) - 273.15}{234.5}\right)\frac{T_S(s) - 273.15}{T_S(s) - 16.01}\right) - \right. \\
& \left. - H_R 611.21 exp\left(\left(18.678 - \frac{T_S(s) - 273.15}{234.5}\right)\frac{T_S(s) - 273.15}{T_S(s) - 16.01}\right)\right]\left(\frac{1}{T_C(s)} - \frac{1}{T_B(s)}\right) \\
& + A_e M(s)\Big[0.0014\big(T_{RES}(s) - T_A(s)\big) + 0.0017(58.7 - \\
& - \frac{1}{133.322}H_R 611.21 exp\left(\left(18.678 - \frac{T_S(s) - 273.15}{234.5}\right)\frac{T_S(s) - 273.15}{T_S(s) - 16.01}\right)\Big]\left(\frac{1}{T_{RES}(s)} - \frac{1}{T_B(s)}\right) + \\
& + \frac{\dot{T}_B(s)}{T_B(s)^2}\int_0^s \left(\varepsilon A_e \sigma_{SB} T_S(r)^4 - \eta A_e \sigma_{SB} T_A(r)^4\right)dr + \\
& + \left(\frac{\dot{T}_B(s)}{T_B(s)^2} - \frac{\dot{T}_S(s)}{T_S(s)^2}\right)1.87 A_e \sqrt{\frac{p(s)}{p_0}}\int_0^s \left(T_S(r) - T_A(r)\right)^{\frac{5}{4}}dr + \\
& + \left(\frac{\dot{T}_B(s)}{T_B(s)^2} - \frac{\dot{T}_C(s)}{T_C(s)^2}\right)A_e \lambda \mu \int_0^s \left[611.21 exp\left(\left(18.678 - \frac{T_S(r) - 273.15}{234.5}\right)\frac{T_S(r) - 273.15}{T_S(r) - 16.01}\right) - \right. \\
& \left. - H_R 611.21 exp\left(\left(18.678 - \frac{T_S(r) - 273.15}{234.5}\right)\frac{T_S(r) - 273.15}{T_S(r) - 16.01}\right)\right]dr + \\
& + \left(\frac{\dot{T}_B(s)}{T_B(s)^2} - \frac{\dot{T}_{RES}(s)}{T_{RES}(s)^2}\right)A_e \int_0^s M(r)\Big[0.0014\big(T_{RES}(r) - T_A(r)\big) + 0.0017(58.7 - \\
& - \frac{1}{133.322}H_R 611.21 exp\left(\left(18.678 - \frac{T_S(r) - 273.15}{234.5}\right)\frac{T_S(r) - 273.15}{T_S(r) - 16.01}\right)\Big]dr - \\
& - \sigma_{O_2}\dot{M}(O_2)(s) + \sigma_{CO_2}\dot{M}(CO_2)(s)\Bigg\}ds - \sigma_{prod,no\,stress}\,t
\end{aligned}
$$

(6)

Assumed approximations are as follows:

$A_e$ = const.
$\dot{\sigma}_{O_2} = 0$
$\dot{\sigma}_{CO_2} = 0$
$M(H_2O_{in}) = 0$
$M(H_2O_{out}) = 0$
$T_{MR} = T_A$

$$\dot{Q}_{prod}(t) = M(t)$$
$$T_{DB} = T_A$$
$$\dot{Q}_{SW}(t) = 0$$
$$w(t) = w = const.$$
$$\sigma prod,no\ stress\ (t) = const.$$

This is not the only possible SEL derived formula, but the above is given by way of a non-limiting example only. Different formulas for calculating SEL can be derived depending on assumed approximations and equations for heat calculations. Alternatively, heat could be measured directly.

When a living subject is twinned, these equations can be used to model the steepness of entropy production that is proportional to the adaptation costs that are assumed to be inter-correlated with stress levels.

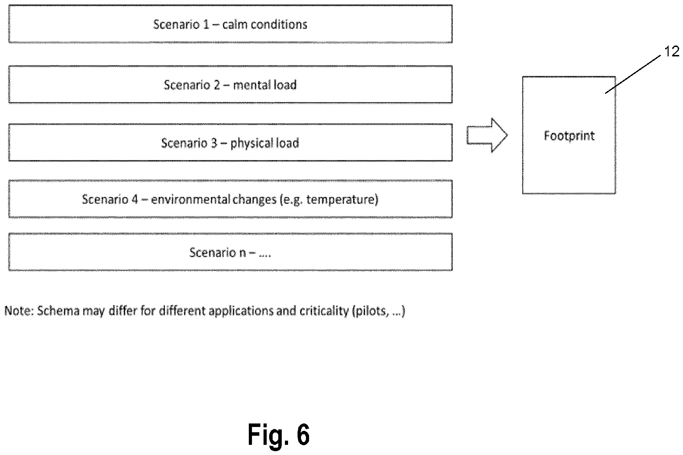

Fig. 6 illustrates standardized scenarios for baseline delta SEL gathering.

The basic hypothesis is that the baseline production of SEL has to be established to make it possible to interpret the data in terms of stress levels. The baseline production of SEL can be evaluated both in a calm scenario (no physical activity, no psychosocial stress or mental effort, non-disturbing environment etc.) or in other scenarios (baseline physical activity, baseline psychosocial stress). The basic hypothesis is that the baseline production of SEL has to be established to make it possible to interpret the data in terms of stress levels. The baseline production of SEL is to be estimated in calm scenario (no physical activity, no psychosocial stress or mental effort, non-disturbing environment etc.). Should such calm scenario not be available, it is possible to approach the approximated baseline production of SEL in other scenarios (baseline physical activity, baseline psychosocial stress). All calculations using these approximated levels of SEL production, however, should be clearly treated as only approximative. Some exemplary scenarios for calculating baseline entropy production rates for obtaining the baseline footprint 12 are shown in Fig. 6.

Most typically, the development of SEL over time is calculated by subtracting two time series, as exemplified above in Eq. (5). The first time series is extrapolated from data from the RMR (Resting Metabolic Rate) subphase. Entropy production during the RMR subphase is extrapolated to the entire duration of the experiment and serves as a baseline entropy production value. The second time series contains data about entropy production from measurement during the entire course of the measurement, including the "stressful" phase, which may be e.g. performing the stressful task, etc.

In an exemplary practical implementation, the RMR subphase can be implemented as follows: The person undergoing baseline measurement are to calm down and relax by letting them delay until they attain their resting metabolic rate RMR defined by i) maximum deviation 10% of oxygen uptake rate, ii) maximum deviation 10% of carbon dioxide liberation rate,) maximum deviation iii) maximum deviation of 5% of respiratory quotient (RQ) continuously during 5 minutes of measurement. The criteria can vary but the basic idea is that if the entropy production is measured when the individuals are as stress-free as possible, reasonably close estimates of the baseline entropy production can be obtained.

Therefore, expected SEL production during the RMR subphase is zero, i.e. $\left(\dfrac{ds_{SEL}}{dt}\right)_{RMR\ subphase} = 0.$

Calculating the slopes of SEL (i.e. average SEL change per unit of time) during the stressful subphase can lead to three scenarios:

$\left(\dfrac{ds_{SEL}}{dt}\right)_{stressful\ subphase} > 0:$ SEL increases with time; positive response in SEL production; SEL production is positive during the stressful subphase of the experiment; SEL increase is associated with stress induction,

$\left(\dfrac{ds_{SEL}}{dt}\right)_{stressful\ subphase} = 0:$ SEL remains constant; no response in SEL production; SEL production is zero during the stressful subphase of the experiment and SEL is thus not associated with stress induction,

$\left(\dfrac{ds_{SEL}}{dt}\right)_{stressful\ subphase} < 0:$ SEL decreases with time; negative response in SEL production; SEL production is negative during the stressful subphase of the experiment and SEL is thus negatively associated with stress induction.

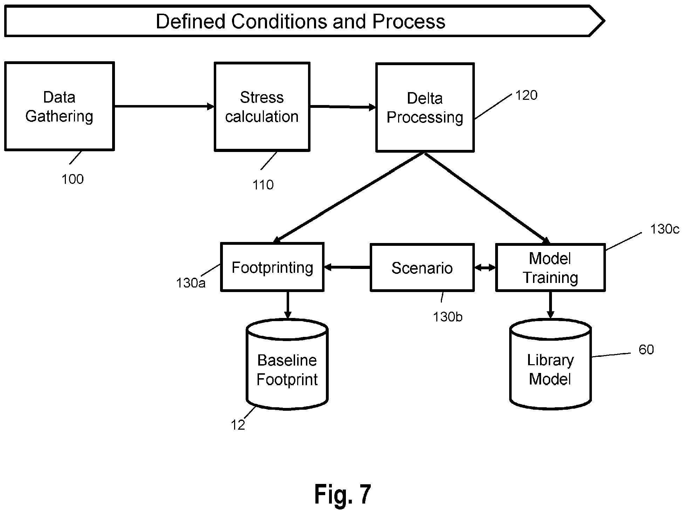

Fig. 7 is a block diagram of the baseline creation process for the twinned individual 21.

Principally, the digital twin 27 needs a numeric representation of a living system stress response to be able to compare the real-time data with the expected data. Real deployment of a digital twin could process and record enough data over the time for training 130c a numeric model using Machine Learning (in the drawing referenced as Library Model 60). Before such a model for an individual 21 can be created, the initial processing of the digital twin 27 is secured by comparison against the baseline footprint 12 of a given living system 21. The baseline footprint 12 is obtained by a defined process ("baseline footprinting" 130a) that results in a baseline digital footprint of SEL changes in given conditions.

Such processes use a set of standardized scenarios 130b as illustrated in Fig. 6.

Fig. 8 is a block diagram of the delta processing 120.

The delta processing comprises algorithmic evaluation of SEL changes computed in time. In detail, the input data are optionally filtered 70 for better evaluation (e.g. fast moving average smoothening, or other suitable filtering algorithm) and used for computation of coefficients and footprint creation, such as statistical coefficients 74 (minimum, maximum, average, standard deviation, etc.), derivatives 76 (steepness of changes coefficients), recovery rates 78 (time from twinned individual being on the same stress level) or similar stress adaptation computed coefficients. These coefficients may be used for footprinting and are therefore summarized as footprint coefficients 75. In addition, the filtered data may be buffered in data buffer 72.

The delta processing 120 is used in both baseline creation process and digital twin evaluation process for preprocessing the SEL changes.

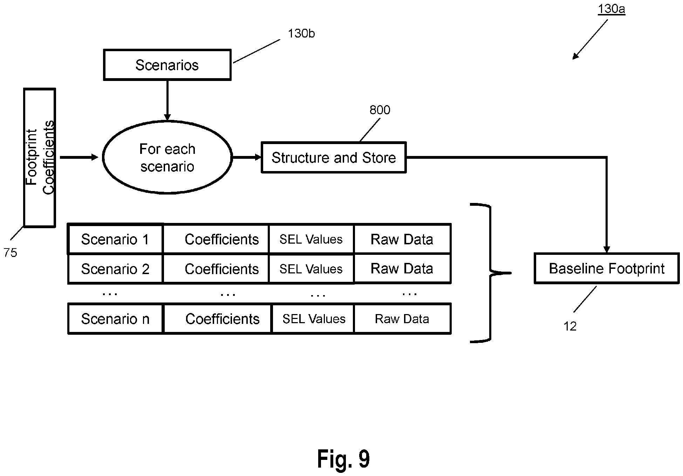

Fig. 9 is a graphic representation of the footprinting process 130a.

The footprinting process 130a stores outputs obtained from the baseline creation process into a set of records 12. These records allow for the digital twin 27 to be initially set up for a given living system 21. For individual categorized scenarios, footprinting process 130a stores all computed coefficients in aggregated and time-series form (coefficients in Fig. 9). Calculated SEL time-series data and raw gathered sensor data are also stored for footprint recalculation in case of equation updates and offline cloud processing, such as advanced library model training using Machine Learning techniques.

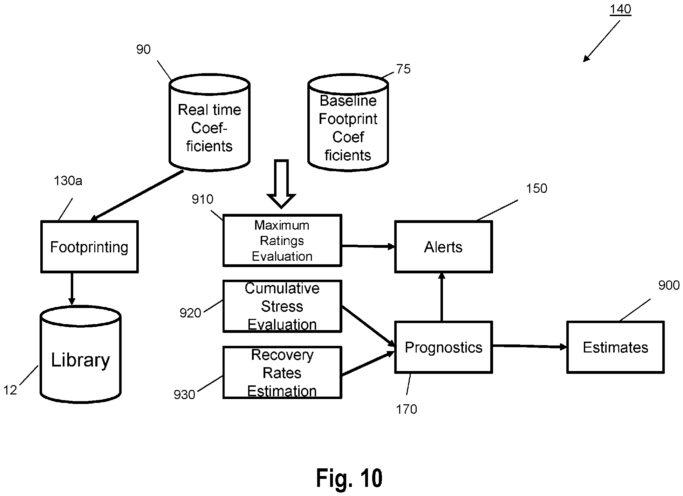

Fig. 10 is a graphic representation of the evaluation process 140 including comparison with the footprint data 12.

The evaluation system generates outputs of the digital twin 27, i.e. alerts 150 or prognostic information 170, such as estimates 900 of future development of the living system 21 or safety time. In one embodiment, the real-time coefficients 90 as well as baseline coefficients 75 present inputs into the evaluation process 140 that can be viewed as a predefined set of rules and algorithms generating alerts 150 or prognostic estimates 170. In embodiments, the evaluation 140 comprises, in particular, maximum ratings evaluation 910, cumulative stress evaluation 920 and recovery rates evaluation 930. Maximum ratings are the condition that should not be exceeded. In the present context, these could refer to values or trajectories of SEL development with a great probability of predetermined adverse events of interest e.g., death, collapse from exhaustion, a significant change in performance etc. Recovery rates in this context refer to the changes of SEL production in given conditions after cessation of the stressful event and/or after intervention. Cumulative stress evaluation refers to the overall SEL production during the whole measurement. The latter two are used as an input for prognostics. An alert 150 can be triggered by both a result of the prognostics 170 and directly by a result of the maximum ratings evaluation 910 in real time.

Digital twin 27 can also be viewed as a continuous real-time data processing system; thus it is desirable to use the gathered and computed data for offline cloud training of a library model of living systems allowing digital twin 27 to have better prognostic 170 and simulation 160 capabilities.

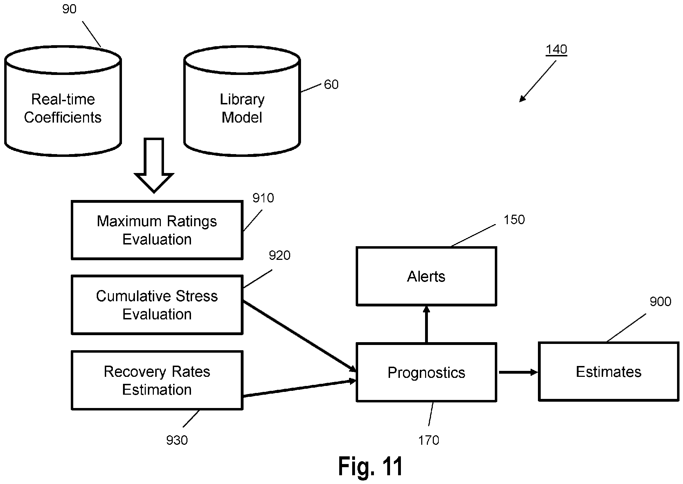

Fig. 11 is a graphic representation of the evaluation process including comparison with the library model 60.

Such evaluation could involve a model 60 trained based on the data gathered during the real deployment of a digital twin 27 on living system 21. In one embodiment, the real-time coefficients 90 as well as library coefficients 60 represent inputs into maximum rating evaluation 910, as well as cumulative stress evaluation 920 and recovery rates estimation, while the latter two can be viewed as inputs into prognostics 170. An alert 150 can be triggered as a result of the prognostics 170, while prognostics also yields estimates 900, while the estimates represent the outputs from the prognostics that are not classified as alert of any kind, but may be used later for more detailed analysis of the scenario.

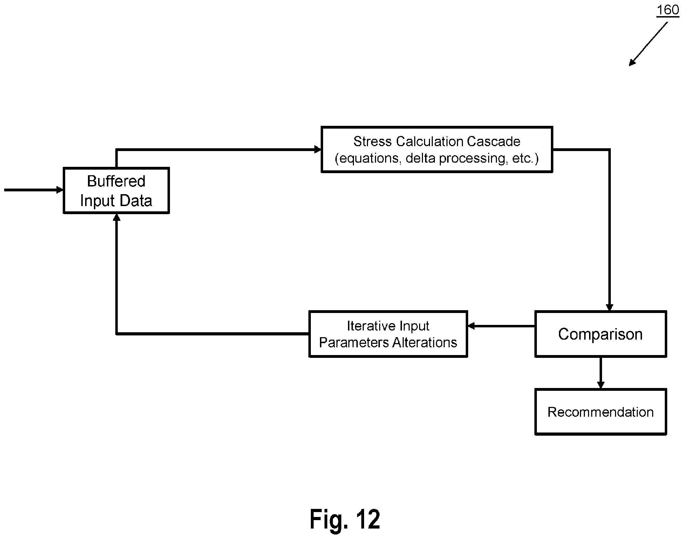

The stress twin can also be used for simulation purposes 160. Essential elements of the simulation process and their inter-relations are illustrated in Fig. 12.

The essence lies in the fact that the whole stress calculation cascade, including stress calculation, delta processing etc., is iteratively repeated. The calculation result which, on the one hand, undergoes threshold comparison for issuing a recommendation or an alert, is, on the other hand, used to iteratively alter calculation input parameters that update the buffered input data. The updated input data from the buffer are then used in the next iteration of stress calculation.

When considering the whole calculation cascade as a one-way process, altering the inputs can be used for simulation purposes that make it possible to re-evaluate the SEL generating scenario with different inputs to observe the possible effects. By inputs, we mean the basic input variables used for SEL calculation that are relevant in given environmental conditions.

The simulation process 160 could in principle also comprise scenarios, when considering the simulation cascade a two-way process, where knowledge of the outcome or a defined desirable outcome may provide inputs for altering of the initial inputs of the SEL calculation. This could, for example, be replacing the simulation process 160 by bifurcation analysis-based models using data gathered in production deployment of a stress twin.

In the following, an example of system functioning using the simulated data will be given and illustrated in Figs. 13 to 19.

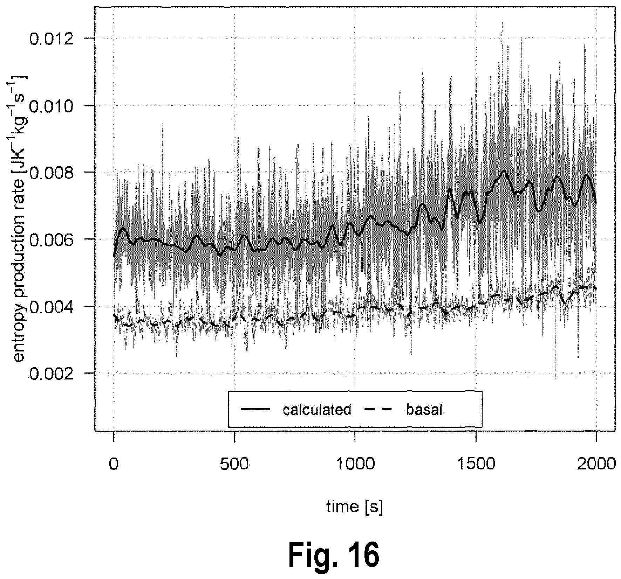

Hereto, we provide a simulation of a human body using the simulated data for a human 170 cm in height, 70 kg in weight, fully naked within a changing environment. Figures 13 to 15 show time series of simulated characteristics, while Figures 16 to 18 show time series of calculated variables of interest (EPR, cEP, SEL rate, SEL).

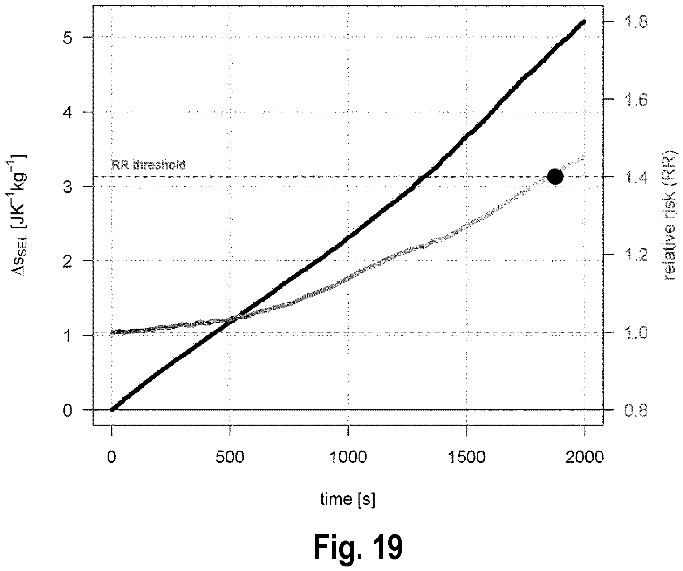

Figure 19 shows an estimation of the risk of an adverse event from the calculated value of SEL utilizing a library of reference data (either personal and/or population data) for given activity and/or adverse events.

The device according to the invention will instruct an operator to attend to the values and will suggest several course of actions, such as immediate cessation of activity or limited continuation or change of activity or change of environmental conditions (such as change of ambient temperature in the room). An alarm will be given after the pre-set value of acceptable risk is exceeded by the "relative risk" function, which is a function of time. The relative risk function for a monitored individual is uniquely determined by the calculated SEL production shown in the bold dashed graph as a function of time and the aforementioned reference data collected in a library. In the drawing, the threshold is indicated as "relative risk (RR) threshold" with a dashed line.

In summary, the present invention relates to the concept of a stress digital twin. The stress digital twin of an individual is a digital, i.e. computer-implemented system, for monitoring a stress level of a living individual on the basis of at least data directly measured on the body of the living individual and by performing a thermodynamic evaluation of the stress entropic load. The stress entropic load is a thermodynamic parameter representing an amount of entropy generated within the living individual due to adaptation to a whole set of environmental influences in a given scenario at a given point of time. Based on a comparison with pre-collected reference data (library data), the invention triggers actions such as withdrawal or replacement of individuals acting in a given scenario or the issuing of alerts.

**Claims**

1.   A system for monitoring a stress level of a living individual (21), the system comprising:

receiving means for receiving data (36) directly measured by a plurality of sensors (30) on the body of the living individual (21);
processing means for evaluating a stress level of the living individual (21) based on the received data (36), wherein the evaluating the stress level includes calculating a stress entropic load, the stress entropic load being a thermodynamic parameter representing an amount of entropy generated within the living individual (21) due to adaptation to a whole set of environmental influences in a given scenario at a given point of time; and
an output interface for generating an output (14) indicating the stress level of the living individual based on the result of the evaluation by said processing means.

2.   A system according to claim 1, wherein said processing means further being adapted to predict the adaptation of the living individual (21) for future points of time, by extrapolation and/or simulation based on the evaluation result.

3.   A system according to claim 2, wherein said processing means further being adapted to predict an estimated time to adaptation failure of the living individual (21) based on said extrapolation and/or simulation, the time to adaptation failure giving a time limit for replacement of the living individual (21) operating in said given scenario.

4.   A system according to claim 3, wherein said estimated time to adaptation failure being based on a comparison of a unique function of a stress entropic load predicted for future points in time by extrapolation and/or simulation with a given threshold.

5.   A system according to claim 4, wherein said unique function of the stress entropic load being determined based on a predetermined library (60) of reference data.

6.   A system according to any of claims 3 to 5, wherein
said processing means further being adapted to determine that the time limit for replacement has been reached, and
said output interface further being adapted to output an indication to replace the living individual (21) in response to a determination by said processing means that the time limit for replacement has been reached.

7.   A system according to any of claims 1 to 6, wherein said receiving means further receiving data directly measured by at least one environment sensor(32) for being used in the evaluation by said processing means.

8.   A system according to any of claims 1 to 7, wherein said calculation comprises the calculation of a stress entropic load change of the living individual (21) during a predetermined time interval, calculated as a difference between the overall entropy production of the living individual calculated based on said data measured by said sensors (30, 32) during said predetermined time interval and an a baseline entropy production of said living individual (21) over said predetermined time interval, said baseline entropy production indicating the entropy production of said living individual (21) in a stress free scenario.

9. A system according to claim 8, wherein said baseline entropy production is calculated in advance, by means of a calculation of the overall entropy production of the living individual (21) during said predetermined time interval in a stress free scenario, based on respective sensor data measured in said stress free scenario.

10. A system according to claim 9, further comprising a database for storing data representing said baseline entropy production for a given living individual (21), as a footprint of said living individual.

11. A system according to claim 8, wherein said baseline entropy production is estimated on the basis of respective data (60) calculated and collected for a representative population of said kind of living individuals (21), by further taking into account individual characteristics of the particular living individual which is monitored.

12. A system according to claim 8 or 11, wherein said baseline entropy production is assumed to have a constant rate.

13. A computer-implemented method of monitoring a stress level of a living individual, the method comprising the steps of receiving (100) data (36) directly measured by a plurality of sensors (30) on the body of the living individual (21), evaluating (140) a stress level of the living individual (21) based on the received data, wherein the evaluating (140) the stress level includes calculating (110) a stress entropic load, the stress entropic load being a thermodynamic parameter representing an amount of entropy generated within the living individual (21) due to adaptation to a whole set of environmental influences in a given scenario at a given point of time; and outputting (200) an output (14) indicating the stress level of the living individual (21) based on the result of the evaluation.

14. A method according to claim 13, further comprising the step of predicting (170) an estimated time (140) to adaptation failure of the living individual (21) by an extrapolation and/or simulation (160) based on the evaluation result, the time to adaptation failure giving a time limit for replacement of the living individual (21) operating in said given scenario.

15. A method according to claim 14, further comprising the step of outputting (150) an indication to replace the living individual (21) in response to a determination that the time limit for replacement has been reached.

27

21

Twinned
Individual

Environ
ment

23

External
Sensor
Data
Source

25

Stress Digital Twin

Twin
Sen
sors

Data
Interface

Twin
Processing

Outputs
Processing

200

# Fig. 1

Fig. 2

Sensor data — 20

Library data — 22

Anthropometric data — 24

Individual Baseline data — 26

Current task data — 28

**Fig. 3**

100

**Data Gathering**

30

32

Human Sensors

Environment Sensors

External Sensor Data Interface

34

Data Normalization

105

SEL calculation

Sensor Data Storage — 36

**Fig. 4**

Fig. 5

Scenario 1 – calm conditions

Scenario 2 – mental load

Scenario 3 – physical load

Scenario 4 – environmental changes (e.g. temperature)

Scenario n – ....

Footprint

12

Note: Schema may differ for different applications and criticality (pilots, ...)

# Fig. 6

Defined Conditions and Process

Data Gathering
100

Stress calculation
110

Delta Processing
120

Footprinting
130a

Scenario
130b

Model Training
130c

Baseline Footprint
12

Library Model
60

Fig. 7

Fig. 8

**Fig. 9**

Fig. 10

90

Real-time Coefficients

Library Model 60

140

Maximum Ratings Evaluation 910

Cumulative Stress Evaluation 920

Recovery Rates Estimation

930

Alerts 150

Prognostics

170

Estimates 900

## Fig. 11

160

Buffered
Input Data

Stress Calculation Cascade
(equations, delta processing, etc.)

Comparison

Iterative Input
Parameters Alterations

Recommendation

Fig. 12

Fig. 13

Fig. 14

Fig. 15

Fig. 16

**Fig. 17**

Fig. 18

Fig. 19

# EUROPEAN SEARCH REPORT

Application Number

EP 20 17 0425

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Sensorik Prof ET AL: "Exzellenzcluster Cognitive Interaction Technology Kognitronik Design and Application of Wireless Body Sensors", , 19 December 2019 (2019-12-19), XP055729493, Retrieved from the Internet: URL:https://pub.uni-bielefeld.de/download/2939716/2939717/20191228_genehmigte_Dissertation_Timm_Hoermann_Book.pdf [retrieved on 2020-09-10] * whole document, in particular 4.3.2.1 and 4.3.4, p.81, table 4.2 and 4.5, fig. 4.5, 4.9 * ----- | 1-15 | INV. G16H50/30 G16H50/50 |
| X | FILIP ZLÁMAL ET AL: "Stress entropic load: New stress measurement method?", PLOS ONE, vol. 13, no. 10, 18 October 2018 (2018-10-18), page e0205812, XP055729483, DOI: 10.1371/journal.pone.0205812 * whole doc, in particular titel, abstract, p.2-4, 12, fig.1 * ----- | 1-15 | |
| X | SATISH BOREGOWDA ET AL: "Measuring Entropy Change in a Human Physiological System", JOURNAL OF THERMODYNAMICS, vol. 2016, 1 January 2016 (2016-01-01), pages 1-8, XP055729737, ISSN: 1687-9244, DOI: 10.1155/2016/4932710 * whole doc, in particular titel and abstract, fig. 1 and table 1 and 2. * ----- -/-- | 1-15 | |

TECHNICAL FIELDS SEARCHED (IPC)

G16H

The present search report has been drawn up for all claims

| Place of search | Date of completion of the search | Examiner |
|---|---|---|
| Munich | 11 September 2020 | Lüdemann, Susanna |

EPO FORM 1503 03.82 (P04C01)

page 1 of 2

Europäisches
Patentamt

European
Patent Office

Office européen
des brevets

**EUROPEAN SEARCH REPORT**

Application Number

EP 20 17 0425

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MARIO SALAI ET AL: "Stress Detection Using Low Cost Heart Rate Sensors", JOURNAL OF HEALTHCARE ENGINEERING, vol. 2016, 1 January 2016 (2016-01-01), pages 1-13, XP055729490, Brentwood ISSN: 2040-2295, DOI: 10.1155/2016/5136705 * the whole document * | 1-15 | |

TECHNICAL FIELDS
SEARCHED      (IPC)

The present search report has been drawn up for all claims

| Place of search | Date of completion of the search | Examiner |
|---|---|---|
| Munich | 11 September 2020 | Lüdemann, Susanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)